Europäisches Patentamt

European Patent Office

Office européen des brevets

(19)

(11) Publication number: 0 495 892 B1

# EUROPEAN PATENT SPECIFICATION

(12)

(45) Date of publication of patent specification: 19.07.95   (51) Int. Cl.⁶: **C07C 17/00**

(21) Application number: **90915920.4**

(22) Date of filing: **09.10.90**

(86) International application number:
**PCT/US90/05637**

(87) International publication number:
**WO 91/05752 (02.05.91 91/10)**

(54) **Halocarbon hydrogenolysis.**

(30) Priority: **10.10.89 US 418832**

(43) Date of publication of application:
**29.07.92 Bulletin 92/31**

(45) Publication of the grant of the patent:
**19.07.95 Bulletin 95/29**

(84) Designated Contracting States:
**DE ES GB IT NL**

(56) References cited:
**US-A- 2 494 064**

**Chemical Abstracts, volume 111, no. 13, Columbus, Ohio, US, Y. Furutaka et al, "Preparation of 1,1,1,2-tetrafluoroethane"; & JP-A-0193549**

(73) Proprietor: **E.I. DU PONT DE NEMOURS AND COMPANY**
**1007 Market Street**
**Wilmington**
**Delaware 19898 (US)**

(72) Inventor: **KIELHORN, Fernando, Frederick**
**303 Weldin Road**
**Wilmington, DE 19803 (US)**
Inventor: **MANOGUE, William, Henry**
**224 Beverly Road**
**Newark, DE 19711 (US)**
Inventor: **RAO, V., N., Mallikarjuna**
**1 Georgetown Avenue**
**Wilmington, DE 19809 (US)**

(74) Representative: **Woodcraft, David Charles et al**
**BROOKES & MARTIN**
**High Holborn House**
**52/54 High Holborn**
**London, WC1V 6SE (GB)**

**Description**

This invention relates to a process for the hydrogenolysis of halocarbons.

At this time there is a desire to produce halocarbons of reduced chlorine content. Hydrogenolysis is a known method for doing this. For example, see UK Patent 1,578,933 which discloses a process for the hydrogenolysis of $CF_3CFHCl$ to $CF_3CH_2F$ using a hydrogenation catalyst, e.g. palladium supported on alumina or carbon. Hydrogenolysis of fluorochlorocarbons by passage through empty tubes made of various materials is also known, e.g. US 2,615,926 discloses platinum tubes, US 2,704,775 discloses nickel or stainless steel tubes and US 3,042,727 discloses a Vycor[(R)] tube.

US-A-2,494,064 describes a process for high temperature hydrogenolysis of multi carbon saturated fluorocarbons. The object of the process is to cleave a carbon-carbon bond in the fluorocarbon and to add hydrogen to the cleaved fluorocarbon fragments. The fluorocarbon is heated with hydrogen in a reaction tube to a temperature of the order of 800 to 900°C which is sufficiently high to bring about the above-mentioned cleavage. Starting materials include saturated fluorocarbon monohydrides, monochlorides and monobromides.

Chem. Abstracts 111 (1989) Sept 25, No. 13, describes the preparation of a tetrafluoroethane in which $CF_3CHClF$ and/or $CF_3CFCl_2$ is heated with hydrogen in the presence of activated carbon at a temperature in the range of 400 to 600°C. There is no disclosure of the nature of the reactor.

It is desired to provide a process for converting a halocarbon to a more hydrogenated form with high selectivity and particularly to provide such a process wherein formation of solids and plugging of reaction vessels is minimized.

We have discovered an improved hydrogenolysis process for reducing the chlorine and/or bromine content of halocarbons. The process may be used for producing saturated halocarbon products such that the yield loss to olefins, coupled by-products, hydrocarbons or fragmented products is less than 10%. The process involves contact a halocarbon of the formula:

$$C_nH_mF_pX_q$$

wherein

X is Cl or Br,
n is 1 to 10,
m is 0 to 20,
p is 0 to 21,
q is 1 to 22, provided that $m + p + q$ equals $2n + 2$ when the compound is acyclic and equals $2n$ when the compound is cyclic, and provided that when n is 1, q is at least 2, with at least 0.1 mole of hydrogen per mole of said halocarbon to effect hydrogenolysis of the halocarbon characterised in that a mixture of hydrogen and said halocarbon is contacted in a reaction vessel of aluminum, molybdenum, titanium, nickel, iron, cobalt, or their alloys or of chromium or of silicon carbide, which is either empty or is packed with particles or formed shapes of aluminum, molybdenum, titanium, nickel, iron, cobalt, or their alloys or silicon carbide or low surface area carbon at a pressure within the range of from 0 to 6900 kPa (0 psig to 1000 psig), at a temperature within the range of from 350°C to 700°C and for a time sufficient to produce as the major product a product of the above formula where at least one of X has been replaced by a hydrogen atom.

The process of the invention further provides improved conversions and selectivity and has the further advantage that it does not produce olefins as the major product. Furthermore, the process minimizes the formation of solids in the reaction vessel, thus permitting long-term operation with less plugging.

An important aspect of the present invention is conducting the hydrogenolysis of halocarbons in the presence of silicon carbide and/or at least one metal selected from aluminum, molybdenum, titanium, nickel, iron, cobalt or their alloys. The metals may be coated on the inside surface of a reaction vessel (e.g., by plating or sputtering the metals or their alloys onto the inside surface). Such coating can help to minimize corrosion of the reaction vessel well. A reaction vessel of these materials (e.g., a metal tube) optionally packed with the metal in suitable form or an inert material such as silica, silicon carbide or low surface area carbon (e.g., shot coke) may also be used. When reference is made to alloys, it is meant a nickel alloy containing from 1 to 99.9% (by weight) nickel, a cobalt alloy containing 1 to 99.9% (by weight) cobalt, an iron alloy containing 0.2 to 99.9% (by weight) iron, a molybdenum alloy containing 70 to 99.9% (by weight) molybdenum, an aluminum alloy containing 80 to 99.9% (by weight) aluminum and a titanium alloy containing 72 to 99.8% (by weight) titanium. Preferably the remainder of these alloys is selected such that the alloy consists essentially of (i) one or more metals selected from aluminum, molybdenum, titanium, nickel, iron and cobalt, and optionally (ii) chromium and/or tungsten.

Most preferred for the practice of this invention are nickel or alloys of nickel such as those containing 52% to 80% nickel, e.g., Inconel® 600 nickel alloy or Hastelloy® C276 alloy.

When used for packing, the metal, alloys or inert material may be particles or formed shapes such as, for example, perforated plates, saddles, rings (e.g., Pall® rings), wire, screen, chips, pipe, shot, gauze and wool. Although an empty reaction vessel (e.g., an empty tube) may be used, the use of this type of packing material can provide the advantage of minimizing backmixing. These types of packing material can also serve as heat transfer materials. In many embodiments, perforated plates, saddles and rings can be especially useful.

The invention is applicable to the hydrogenolysis of halocarbons. The halocarbons can contain 1 to 10 carbon atoms, preferably 1 to 4 carbon atoms, most preferably 1 to 3 carbon atoms. The halocarbons include cyclic as well as acyclic compounds and can be generically represented by the empirical formula $C_nH_mF_pX_q$, where X is Cl and/or Br, preferably Cl, and n is an integer from 1 to 10, m is an integer from 0 to 20, p is an integer from 0 to 21, and q is an integer from 1 to 22, provided that $m + p + q = 2n + 2$ when the compound is acyclic and equals 2n when the compound is cyclic. For single carbon compounds (i.e., n is 1) the invention is particularly applicable when q is at least 2.

In a preferred embodiment the halocarbons are represented by the above empirical formula where n = 1 to 4, m is 0 to 8, p is 0 to 9, and q is 1 to 9. Preferably, when n is 2 or more, p is at least 1.

The above halocarbons are either commercially available or can be prepared by known methods or adaptation of known methods.

As previously indicated these starting materials when subjected to the process of the invention will result in products wherein one or more X (e.g., chlorine) has been replaced by hydrogen. Thus the products of the hydrogenolysis reactions of the $C_1$ halocarbons will contain one or two hydrogen atoms, preferably one, and those from $C_2$ compounds from one to three hydrogen atoms, preferably one to two. The $C_3$ halocarbons hydrogenolysis products will contain one to five hydrogen atoms with those containing one to four being preferred. In a similar manner the $C_4$ to $C_{10}$ halocarbon products will contain one or more hydrogen atoms. The preferred process of this invention does not produce olefins as the major product. Instead, the major product of the conversion is a hydrogenolysis product wherein at least one X of the halocarbon starting material has been replaced by a hydrogen atom. This is particularly important for the hydrogenolysis of halocarbons where n is 2 to 10 (i.e., multicarbon halocarbons) where such factors as olefin production can be of concern at temperatures of 350°C or more. For example, $CF_3CCl_2F$ can be converted with high selectivity to a hydrogenolysis product consisting primarily of $CF_3CHClF$ and $CF_3CH_2F$ with very little olefin formulation. In a preferred embodiment of this invention using halocarbons containing fluorine and chlorine, at least 90% of the hydrogenolysis products contain the same number of fluorines as the original halocarbon. Furthermore the yield loss to olefins, coupled by-products, hydrocarbons, fragmentation products or carbon is less than 10%.

Examples of olefins are products such as $CClF=CCF_2$ or $CF_2=CF_2$ the former of which can be obtained from hydrogenolysis of $CCl_2FCClF_2$ and the latter from hydrogenolysis of $CClF_2CClF_2$. An example of a coupled by-product is $CF_3CF=CFCF_3$ which can be obtained by the hydrogenolysis of $CClF_2CClF_2$. Examples of hydrocarbon products are $CH_4$, $C_2H_6$ and $C_3H_8$ which can be obtained by the hydrogenolysis of $CCl_2F_2$, $CCl_2FCClF_2$ and $CF_3CClFCF_3$ respectively. Examples of fragmentation products are $CF_3H$ and $CH_2F_2$ which can be obtained by the hydrogenolysis of $CF_3CCl_2F$ and its isomer.

The reaction temperature can range from 350°C to 700°C. Preferably the reaction temperature is at least about 400°C.

The amount of hydrogen contained in the gas stream contacted with the gaseous halocarbon should be at least 0.1 mole per mole of halocarbon. Hydrogen amounts ranging from 0.2 to 5 moles per mole of halocarbon are used for some embodiments. In general, the amount of hydrogen preferably ranges from 0.2 to 60 moles per mole of halocarbon and more preferably ranges from 0.4 to 40 moles per mole of halocarbon. The hydrogen can be fed either in the pure state or diluted with an inert gas, e.g., nitrogen, helium, or argon.

The process pressure is operable over a broad range of pressures. Generally atmospheric (i.e., 0 psig) or superatmospheric pressures of up to 6900 kPa (1000 psig) are employed. Preferably the pressure is at least about 172 kPa (25 psig).

The extent of the replacement of halogen by hydrogen increases with reaction time. Reaction times between 0.1 minutes and 25 minutes are preferred. Most preferred are reaction times between 0.2 and 8 minutes.

An important feature of the process of the invention is that through selection of the appropriate metal and process conditions, a desired halocarbon hydrogenolysis product can be obtained as the major product with high selectivity and minimal formation of unwanted by-products. Preferably the reaction time and temperature are selected to obtain long term (>1000 hours) plug free operation and to provide as the major product of the conversion hydrogenolysis product which retains the fluorine content of the starting halocarbon while at least one X is replaced by hydrogen. In many embodiments the reaction time and temperature are controlled so that at least about 90% of halocarbon converted has the same number of fluorine atoms as the halocarbon starting material. Also, in many embodiments the combined yield losses to olefins, coupled by-products, hydrocarbons, or fragmentation products is less than 10%.

An additional desirable feature is that through a selection of an appropriate reaction vessel and packing (e.g., metals, alloys, or inert materials) and process conditions, the products of the hydrogenolysis can contain in high selectivity just one less chlorine or bromine than was present in the starting material. This is particularly useful when q is 2 or more, and it is desired to obtain a major product of the conversion, hydrogenolysis product which contains chlorine and/or bromine. For example, starting with a one-carbon compound containing two or more chlorine or bromine atoms, products containing just one less chlorine or bromine can be obtained in high selectivity.

Although substantial conversions can be achieved in a once-through system, recycle of unreacted halocarbons or intermediates can be employed in a conventional manner. The processes of this invention are considered to be characterized by relativity high activation energies when compared to catalytic hydrogenolysis over conventional Pd/C catalyst. For example, the activation energy for the hydrogenolysis of $CF_3CCl_2F$ over a 0.5% Pd/C catalyst at 167°C to 200°C was found to be 14-17 Kcal/mole. The activation energy for the hydrogenolysis of $CF_3CHClF$ over a 0.5% Pd/C catalyst at 249°C to 288°C was found to be 22-28 Kcal/mole. In contrast, the activation energies for the hydrogenolysis reactions of these compounds conducted in the reaction vessels of this invention, either empty or packed, were found to be considerably larger as exemplified in Table A.

TABLE A

| Activation Energy Data High Temperature Hydrogenolysis | | | |
|---|---|---|---|
| Feed | Temp. Range | Packing | Activation Energy |
| F114$_a$[1] | 450-550°C | - | 49±3 Kcal/mole |
| F114$_x$[2] | 440-600°C | - | 47±2 |
| F124[3] | 510-600°C | - | 49±7 |
| F114$_x$ | 400-570°C | shot coke | 35±1 |
| F114$_x$ | 400-500°C | nickel screen | 34±3 |
| F124 | 510-570°C | Inconel® screen | 41±3 |
| F124 | 520-580°C | shot coke | 37±1 |

[1]F114$_a$ = $CF_3CCl_2F$
[2]F114$_x$ = Du Pont commercial $CClF_2CClF_2$ containing some $CF_3CCl_2F$
[3]F124 = $CF_3CHClF$

The products of the reaction can be separated and purified by conventional means. The products can be used as solvents, blowing agents, refrigerants and propellants.

Practice of the invention will become further apparent from the following non-limiting examples. In the following Examples the following general procedure was employed, unless otherwise indicated.

General Procedure - A flow reactor under microprocessor control was used. The reactor, unless otherwise indicated, was a 380 x 6.35 mms (15" x 1/4") o.d. or 9.5 mm (3/8") o.d. Inconel® 600 nickel alloy tube or a 380 x 9.5 mm (15" x 3/8") Hastelloy® C276 nickel alloy tube bent into a U shape and immersed in a heated fluidized sand bath for temperature control. Inconel® 600 is a commercial alloy containing 76% nickel, 15.5% chromium and 8% iron. Hastelloy® C-276 is a commercial alloy containing 59% nickel, 15.5% chromium, 16% molybdenum and 3.75% tungsten.

The reactor was used either empty or filled with various packing materials as described in the Examples. Hydrogen gas was metered into the system through mass flow controllers. Liquid halocarbons were fed from a syringe pump and vaporized before entering the reactor. Conversions and yields were measured by taking gas stream samples into a gas chromatograph. Product identification was by gc retention times with confirmation by gc-mass spectrometer analysis of samples.

4

EXAMPLE 1

$$CF_3CCl_2F + H_2 ----> CF_3CHClF + CF_3CH_2F$$

2,2-Dichloro-1,1,1,2-tetrafluoroethane (1.47 g/hr) and hydrogen (molar ratio of $H_2/CF_3CCl_2F$ = 1.9) were fed into the 6.35 mm (1/4") empty Inconel® nickel alloy reactor for 38 hours at 450°-550°C and 250 psig. A sample taken after 14 hours at 550°C showed an 89% conversion of $CF_3CCl_2F$ with a 65% selectivity to $CF_3CHClF$ and a 32% selectivity to $CF_3CH_2F$. Overall selectivity to the two products was 97%.

EXAMPLE 2

$$CF_3CCl_2F + H_2 ----> CF_3CHClF + CF_3CH_2F$$

2,2-Dichloro-1,1,1,2-tetrafluoroethane (1.47 g/hr) and hydrogen (molar ratio of $H_2/CF_3CCl_2F$ = 1.9) were fed into the 6.35 mm (1/4") empty Inconel® nickel alloy reactor for 132 hours at 350°-550°C and 250 psig. At 350°C a 2.3% conversion of $CF_3CCl_2F$ with a 76% selectivity to $CF_3CHClF$ and $CF_3CH_2F$ was observed. A sample taken after 20 hours at 500°C showed an 83% conversion of $CF_3CCl_2F$ with a 98% selectivity to $CF_3CHClF$ and $CF_3CH_2F$.

EXAMPLE 3

$$CF_3CHClF + H_2 ----> CF_3CH_2F$$

2-Chloro-1,1,1,2-tetrafluoroethane (1.0 g/hr) and hydrogen (molar ratio of $H_2/CF_3CCl_2F$ = 4.9) were fed into the 6.35 mm (1/4") empty Inconel® nickel alloy reactor for 7 hours at 550°C and 250 psig with average $CF_3CHClF$ conversions of 86% with 98% selectivity to $CF_3CH_2F$ and 0.4% selectivity to $CF_3CH_3$.

EXAMPLE 4

$$CF_2Cl_2 + H_2 ----> CF_2HCl$$

Dichlorodifluoromethane (9.0 g/hr) and hydrogen (molar ratio of $H_2/CF_2Cl_2$ = 1.0) were fed into the 6.35 mm (1/4") empty Inconel® nickel alloy reactor as described above for 89 hours at 300 psig, including 79 hours at 500-550°C. For 12 hours at 500°C during this run, at a mean age of 64 synthesis hours, the average conversion of $CF_2Cl_2$ was 26%.

EXAMPLE 5

$$CF_3CClFCF_3 + H_2 ----> CF_3CHFCF_3$$

2-Chloroheptafluoropropane (1.5 g/hr) and hydrogen (22 cc/min) were fed into the 6.35 mm (1/4") empty Inconel® nickel alloy reactor for 3 hours at 450°C and 250 psig with 30-40% conversion and a 98% selectivity to $CF_3CHFCF_3$.

EXAMPLE 6

$$CF_3CClFCF_3 + H_2 ----> CF_3CHFCF_3$$

2-Chloroheptafluoropropane (1.38 g/hr) and hydrogen (22 cc/min) were fed into the 6.35 mm (1/4") Inconel® nickel alloy reactor filled with Inconel® nickel alloy chips (10 g). Operation at 500°C and 250 psig for 33.3 hours gave an average of 91.3% conversion with 99.4% selectivity to $CF_3CHFCF_3$.

EXAMPLE 7

$$CF_3CCl_2F + H_2 ----> CF_3CHClF + CF_3CH_2F$$

2,2-Dichloro-1,1,1,2-tetrafluoroethane (2.9 or 5.9 g/hr) and hydrogen (molar ratio of $H_2/CF_3CCl_2F$ = 2.2 or 4.3) were fed into the 9.5 mm (3/8") Inconel® nickel alloy reactor filled with Inconel® nickel alloy wool (7.96 g) for 106 hours at 400-500°C and 250 psig. The average conversion of $CF_3CCl_2F$ over the whole period was 99.9%. For a 12-hour period at 450°C with a $CF_3CCl_2F$ feed rate of 5.9 g/hr (molar ratio $H_2/CF_3CCl_2F$ = 4.3) the following average selectivities were observed: 69% $CF_3CHClF$ and 26% $CF_3CH_2F$.

EXAMPLE 8

$CF_3CHClF + H_2 \longrightarrow CF_3CH_2F$

2-Chloro-1,1,1,2-tetrafluoroethane (5.5 g/hr) and hydrogen (molar ratio of $H_2/CF_3CHClF$ = 1.1) were fed into the 9.5 mm (3/8") Inconel® nickel alloy reactor filled with a pure nickel screen (8.77 g), operated at various feed rates and a pressure of 300 psig. With a $CF_3CHClF$ feed rate of 5.48 g/hr and hydrogen (molar ratio of $H_2/CF_3CHClF$ = 1.1) conversion at 525°C and an average synthesis time of 82 hours averaged 47% with 98% selectivity to $CF_3CH_2F$ for 12 hours. At an average synthesis time of 644 hours conversion averaged 39% with 97% selectivity to $CF_3CH_2F$. At 1181 synthesis hours the operating pressure was increased to 500 psig. Conversion of $CF_3CHClF$ averaged 68% for 14 hours with a selectivity to $CF_3CH_2F$ of 98%.

EXAMPLE 9

$CF_3CHClF + H_2 \longrightarrow CF_3CH_2F$

2-Chloro-1,1,1,2-tetrafluoroethane (2.7 or 5.5 g/hr) and hydrogen (molar ratio of $H_2/CF_3CHClF$ = 1.9) were fed into the 9.5 mm (3/8") Inconel® nickel alloy reactor filled with Inconel® nickel alloy wool (7.96 g) for 23 hours at 400-500°C and 250 psig. Between 18 and 23 hours, at 400°C with a $CF_3CHClF$ feed rate of 2.7 g/hr, the average conversion was 23% and the selectivity to $CF_3CH_2F$ was 82%.

EXAMPLE 10

$CF_2Cl_2 + H_2 \longrightarrow CF_2HCl$

Dichlorodifluoromethane (4.5 or 33.0 g/hr) and hydrogen (molar ratio of $H_2/CF_2Cl_2$ = 1 or 0.5) were fed into the 9.5 mm (3/8") Inconel® nickel alloy reactor filled with pure nickel screen (17.5 g) for 135 hours at 300 psig. For 12 hours at 450° during this run, at a mean synthesis time of 78 hours, with a $CF_2Cl_2$ feed rate of 4.5 g/hr (molar ratio $H_2/CF_2Cl_2$ = 1.0), the average conversion of $CF_2Cl_2$ was 34%.

EXAMPLE 11

$CF_3CClF_2 + H_2 \longrightarrow CF_3CHF_2$

Chloropentafluoroethane vapor (6 cc/min) and hydrogen (5 cc/min) were fed into a Hastelloy® nickel alloy reactor 152 x 12.7 mm OD (6" x 1/2" O.D.) filled with pure nickel screen (39.68 g) at 550°C and atmospheric pressure. The reaction products were analyzed with the following results: 59% conversion of $CF_3CClF_2$ with a 97% selectivity to $CF_3CHF_2$.

EXAMPLE 12

$CF_3CClF_2 + H_2 \longrightarrow CF_3CHF_2$

Chloropentafluoroethane vapor (5 cc/min) and hydrogen (6 cc/min) were fed into an Inconel® nickel alloy reactor, 152 x 12.7 mm OD, (6" x 1/2" O.D.) filled with pure nickel screen (51.98 g) at 550°C and atmospheric pressure. The reaction products were analyzed with the following results: 65% conversion of $CF_3CClF_2$ with a 95% selectivity to $CF_3CHF_2$.

The reaction was run under the same conditions as described above, except that the feed rates were changed to $CF_3CClF_2$ (5 cc/min) and $H_2$ (12 cc/min). The reaction products were analyzed with the following results: 62% conversion of $CF_3CClF_2$ with an 86% selectivity to $CF_3CHF_2$.

EXAMPLE 13

$CClF_2CClF_2/CF_3CCl_2F + H_2 ---- CHF_2CClF_2/CF_3CHClF + CHF_2CHF_2/CF_3CH_2F$

A vapor mixture of $CClF_2CClF_2(9)/CF_3CCl_2F(1)$ (5 cc/min) and hydrogen (6 cc/min) was fed into a Hastelloy® nickel alloy reactor, 152 x 12.7 mm OD, (6" x 1/2" O.D.) filled with pure nickel screen (39.68 g) at 550°C and atmospheric pressure. The reaction products were analyzed with the following results: 61% conversion of $CClF_2CClF_2/CF_3CCl_2F$ with a 46% selectivity to $CHF_2CClF_2/CF_3CHClF$ and a 34% selectivity to $CHF_2CHF_2/CF_3CH_2F$.

EXAMPLE 14

$CF_3CCl_2F + H_2 ---> CF_3CHClF + CF_3CH_2F$

2,2-Dichlorotetrafluoroethane vapor (5 cc/min) and hydrogen (6 cc/min) were fed into an Inconel® nickel alloy reactor, 152 x 12.7 mm OD, (6" x 1/2" O.D.) filled with pure nickel screen (51.98 g) at 550°C and atmospheric pressure. The reaction products were analyzed with the following results: 83% conversion of $CF_3CCl_2F$ with a 5% selectivity to $CF_3CHClF$ and a 66% selectivity to $CF_3CH_2F$.

EXAMPLE 15

$CF_3CHClF + H_2 ---> CF_3CH_2F$

2-Chloro-1,1,1,2-tetrafluoroethane vapor (5 cc/min) and hydrogen (6 cc/min) were fed into an Inconel® nickel alloy reactor, 152 x 12.7 mm OD, (6" x 1/2" O.D.) filled with pure nickel screen (51.98 g) at 550°C and atmospheric pressure. The reaction products were analyzed with the following results: 58% conversion of $CF_3CHClF$ with an 85% selectivity to $CF_3CH_2F$.

EXAMPLE 16

$CClF_2CCl_2F + H_2 ---> CHClFCClF_2 + CHClFCHF_2$

1,1,2-Trichloro-1,2,2-trifluoroethane (3.13 g/hr) and hydrogen (molar ratio = 4.75) were fed into the 9.5 mm (3/8") Inconel® nickel alloy U-tube reactor as described in the general procedure, with the exit leg filled with pure nickel screen (8 g), at 450°C and 3450 kPa (500 psig). Over a 6-hour period the reaction products were analyzed with the following results: 81% conversion of $CCl_2FCClF_2$ with 96% combined selectivity to $C_2H_3F_3$, $C_2H_2ClF_3$, and $C_2HCl_2F_3$. Selectivity to $CClF = CF_2$ was 2%. When the temperature was raised to 475°C for 7 hours, the average conversion of $CCl_2FCClF_2$ was 97% with 95% combined selectivity to $C_2H_3F_3$, $C_2H_2ClF_3$, and $C_2HCl_2F_3$. Selectivity to $CClF = CF_2$ was 1%.

EXAMPLE 17

$CF_3CHClF + H_2 ---> CF_3CH_2F$

2-Chloro-1,1,1,2-tetrafluoroethane (2.7 g/hr) and hydrogen (molar ratio of $H_2/CF_3CHClF = 0.2$) were fed into an empty, 152 x 12.7 mm OD, (6" x 1/2" O.D.) Hastelloy® C276 nickel alloy reactor for 8 hours at 535°C and 2070 kPa (300 psig). The average conversion of $CF_3CHClF$ was 22% with an average 97% $CF_3CH_2F$ selectivity.

EXAMPLE 18

$CF_3CHClF + H_2 ---> CF_3CH_2F$

2-Chloro-1,1,1,2-tetrafluoroethane (2.7 g/hr) and hydrogen (molar ratio of $H_2/CF_3CHClF = 1.5$) were fed into, 152 x 12.7 mm OD, (6" x 1/2" O.D.) Hastelloy® C276 nickel alloy reactor containing 14/20 mesh acid-washed SiC (6.5 g) for 60 hours at 535°C and 2070 kPa (300 psig). The average conversion of $CF_3CHClF$ was 75% with an average 97% $CF_3CH_2F$ selectivity.

7

EXAMPLE 19

$CF_3CHClF + H_2 ----> CF_3CH_2F$

2-Chloro-1,1,1-tetrafluoroethane and hydrogen were fed at various rates over 113 hours into the 9.5 mm (3/8") Hastelloy® C276 nickel alloy tube operated at 300 psig and containing Conoco Shot coke (9.2 grams, 10 cc), a highly fused petroleum coke with a surface area of 0.5 sq m/g. For an 8-hour period at 560°C and an average time in synthesis of 102 hours, with a $CF_3CHClF$ feed rate of 11.0 g/hr and a hydrogen feed rate of 32 cc/min (molar ratio of $H_2/CF_3CHClF$ = 1) the average conversion of $CF_3CHClF$ was 13% with an average selectivity to $CF_3CH_2F$ of 99%.

EXAMPLE 20

$CClF_2CClF_2 + H_2 ----> CHF_2CClF_2 + CHF_2CHF_2$

Commercial 1,2-dichloro-1,1,2,2-tetrafluoroethane, containing 9% (molar) 1,1-dichloro-1,2,2,2-tetrafluoroethane, and hydrogen were fed at various rates over 150 hours into the 9.5 mm (3/8") Inconel® nickel alloy tube operated at 2070 kPa (300 psig) and containing Conoco Shot coke (9.2 grams, 10 cc), a highly fused petroleum coke with a surface area of 0.5 sq. m/g. For a 16-hour period at 550°C and an average time in synthesis of 59 hours, with a $CClF_2CClF_2$ feed rate of 5.9 g/hr and a hydrogen feed rate of 28 cc/min (molar ratio of $H_2/CClF_2CClF_2$ = 2) the average conversion of the $C_2Cl_2F_4$ isomers was 84% with an average selectivity to $CHF_2CClF_2$ and its isomer of 49%, and an average selectivity to $CHF_2CHF_2$ and its isomer of 47%.

EXAMPLE 21

$CF_3CCl_2F + H_2 ----> CF_3CHClF + CF_3CH_2F$

2,2-Dichloro-1,1,1-tetrafluoroethane (2 mL/h), which was vaporized before being mixed with hydrogen (13 cc/min), was fed into a Hastelloy® C nickel alloy reactor, 152 x 12.7 mm OD, (6" x 1/2" O.D.) as described above, containing Conoco Shot coke (14.0 grams, 10 mesh), a highly fused petroleum coke with a surface area of 0.5 sq m/g at 550°C and 690 kPa (100 psig). After 28 hours of operation, product analysis indicated that $CF_3CCl_2F$ conversion was quantitative and selectivity to $CF_3CHClF$ and $CF_3CH_2F$ was 64.7% and 33.3% respectively.

EXAMPLE 22

$CF_3CClF_2 + H_2 ----> CF_3CHF_2$

Chloropentafluoroethane vapor (10 cc/min) and hydrogen (10 cc/min) were fed into a Hastelloy® C nickel alloy reactor, 152 x 12.7 mm OD, (6" x 1/2" O.D.) as described above, charged with Conoco Shot coke (14.0 grams, 10 mesh), a highly fused petroleum coke with a surface area of 0.5 sq m/g at 550°C. After 10 hours of operation, product analysis indicated that $CF_3CClF_2$ conversion was 7.5% and selectivity to $CF_3CHF_2$ was 94.7%.

This experiment was substantially repeated except that the $CF_3CClF_2$ flow was 5 cc/min and the hydrogen flow was 6 cc/min. Product analysis indicated that $CF_3CClF_2$ conversion was 13.3% and selectivity to $CF_3CHF_2$ was 89.6%.

EXAMPLE 23

$CClF_2CClF_2 + H_2 ---> CHF_2CClF_2 + CHF_2CHF_2$

Commercial 1,2-dichloro-1,1,2,2-tetrafluoroethane, containing 9% (molar) 1,1-dichloro-1,2,2,2-tetrafluoroethane, and hydrogen were fed at various rates for 172 hours into an empty, 380 x 9.5 mm (15" x 3/8" O.D). Hastelloy® C276 nickel alloy tube, as described above, operated at 3450 kPa (500 psig). For a 13 hour period at 500°C and an average time in synthesis of 66 hours, with a $CClF_2CClF_2$ feed rate of 5.9 g/hr and hydrogen feed rate of 10 cc/min (molar ratio of $H_2/CClF_2CClF_2$ = 0.7) the average conversion of $C_2Cl_2F_4$ isomers was 58% with an average selectivity to $CHF_2CClF_2$ and its isomer of 75% and an average

selectivity to $CHF_2CHF_2$ and its isomer of 24%. For a 9 hour period at 500°C and an average time in synthesis of 148 hours, with a $CClF_2CClF_2$ feed rate of 1.47 g/hr and a molar feed ratio of $H_2/CClF_2CClF_2$ of 1.5 the average conversion of $C_2Cl_2F_4$ isomers was 88% with an average selectivity of $CHF_2CClF_2$ and its isomer of 45% and an average selectivity to $CHF_2CHF_2$ and its isomer of 54%.

EXAMPLE 24

$CClF_2CClF_2$ + $H_2$ ---> $CHF_2CClF_2$ + $CHF_2CHF_2$

Commercial 1,2-dichloro-1,1,2,2-tetrafluoroethane, containing 9% (molar) 1,1-dichloro-1,2,2,2-tetrafluoroethane, and hydrogen were fed at various rates for 192 hours into a 380 x 9.5 mm (15" x 3/8") O.D. Inconel® 600 nickel alloy tube, as described above, containing 8.0 g of 24 x 100 mesh nickel screen and operated at 3450 kPa (500 psig). For a 10 hour period at 400°C and a $CClF_2CClF_2$ feed rate of 0.7 g/hr and hydrogen feed rate of 1.7 cc/min (molar ratio of $H_2/CClF_2CClF_2$ = 1); the average conversion of $C_2Cl_2F_4$ isomers was 61% with an average selectivity to $CHF_2CClF_2$ and its isomer of 77.0% and an average selectivity to $CHF_2CHF_2$ and its isomer of 22.7%.

EXAMPLE 25

$CF_3CCl_2F$ + $H_2$ ---> $CF_3CHClF$ + $CF_3CH_2F$

2,2-Dichloro-1,1,1,2-tetrafluoroethane and hydrogen were fed at various rates for 68 hours to an empty Hastelloy® C276 nickel alloy tube, as described above, operated at 3450 kPa (500 psig). For a five hour period at 500°C and an average time in synthesis of 41 hours, with a $CF_3CCl_2F$ feed rate of 5.9 g/hr and a hydrogen rate of 14 cc/min (molar ratio of $H_2/CF_3CCl_2F$ = 1), the average conversion was 64% with an average selectivity to $CF_3CHClF$ of 83.6 and an average selectivity of $CF_3CH_2F$ of 15.6%.

EXAMPLE 26

$CClF_2CClF_2$ + $H_2$ ---> $CHF_2CClF_2$ + $CHF_2CHF_2$

Chrome-Plated Reactor

Commercial 1,2-dichloro-1,1,2,2 tetrafluoroethane, containing 9% (molar) 1,1-dichloro-1,2,2,2 tetrafluoroethane, and hydrogen were fed at various rates over 55 hours into a 380 x 6.35 mm (15" x 1/4") o.d. empty chrome-plated U-tube reactor, as described above, operated at 2070 kPa (300 psig). For a 20-hour period at 500°C and an average time in synthesis of 16 hours, with a $CClF_2CClF_2$ feed rate of 2.9 g/hr and a hydrogen feed rate of 13.3 cc/min (molar ratio $H_2/CClF_2CClF_2$ = 2); the average conversion of the $CClF_2CClF_2$ isomers was 56% with an average selectivity to $CHF_2CClF_2$ and its isomer of 21%, and an average selectivity to $CHF_2CHF_2$ and its isomer of 76%.

EXAMPLE 27

$CClF_2CClF_2$ + $H_2$ ---> $CHF_2CClF_2$ + $CHF_2CHF_2$

Aluminum Reactor

Commercial 1,2-dichloro-1,1,2,2 tetrafluoroethane, containing 9% (molar) 1,1-dichloro-1,2,2,2 tetrafluoroethane, and hydrogen were fed at various rates over 31 hours into an empty 380 x 6.35 mm (15" x 1/4") o.d. aluminum U-tube reactor, as described above, operated at 345 kPa (50 psig). For a 3-hour period at 500°C and an average time in synthesis of 28 hours, with a $CClF_2CClF_2$ feed rate of 1.47 g/hr and a hydrogen feed rate of 7.0 cc/min (molar ratio $H_2/CClF_2CClF_2$ = 2); the average conversion of the $CClF_2CClF_2$ isomers was 5% with an average selectivity to $CHF_2CClF_2$ and its isomer of 49%, and an average selectivity to $CHF_2CHF_2$ and its isomers of 33%.

EXAMPLE 28

$$CClF_2CClF_2 + H_2 \longrightarrow CHF_2CClF_2 + CHF_2CHF_2$$

Titanium Reactor

Commercial 1,2-dichloro-1,1,2,2 tetrafluoroethane, containing 9% (molar) 1,1-dichloro-1,2,2,2-tetrafluoroethane, and hydrogen were fed at various rates over 42 hours into a 380 x 6.35 mm (15" x 1/4") o.d. empty titanium U-tube reactor, as described above, operated at 345 kPa (50 psig). For a 17-hour period at 500°C and an average time in synthesis of 9.5 hours, with a $CClF_2CClF_2$ feed rate of 2.9 g/hr and a hydrogen feed rate of 13.9 cc/min (molar ratio $H_2/CClF_2CClF_2 = 2$) the average conversion of the $CClF_2CClF_2$ isomers was 14.2% with an average selectivity to $CHF_2CClF_2$ and its isomer of 57%, and an average selectivity to $CHF_2CHF_2$ and its isomer of 24.4%.

EXAMPLE 29

$$CF_3CHClF + H_2 \longrightarrow CF_3CH_2F$$

Silicon Carbide Reactor

2-Chloro-1,1,1,2 tetrafluoroethane and hydrogen were fed at various rates over 47 hours into a 380 x 6.35 mm (15" x 1/4") o.d. empty silicon carbide straight tube reactor, as described above, operated at 0 psig. For a 12-hour period at 600°C and an average time in synthesis of 29 hours, with a $CF_3CHClF$ feed rate of 2.74 g/hr and a hydrogen feed rate of 8.2 cc/min (molar ratio $H_2/CF_3CHClF = 1$); the average conversion of the $CF_3CHClF$ was 4.3%, with an average selectivity to $CF_3CH_2F$ of 89.7%.

EXAMPLE 30

$$CF_3CCl_2F + H_2 \longrightarrow CF_3CHClF + CF_3CH_2F$$

Silicon Carbide Reactor

2,2-Dichloro-1,1,1,2-tetrafluoroethane 2.94 g/hr were fed into a silicon carbide straight tube reactor, as described above, with 6.4 cc/min of hydrogen (molar ratio $H_2/CF_3CCl_2F = 1$) operated at 0 psig for 41 hours. Over a 15-hour period at 500°C and an average time in synthesis of 27 hours; the average conversion of $CF_3CCl_2F$ was 23% with an average selectivity to $CF_3CHClF$ of 54% and an average selectivity to $CF_3CH_2F$ of 0.6%.

EXAMPLE 31

$$CClF_2CClF_2 + H_2 \longrightarrow CHF_2CClF_2 + CHF_2CHF_2$$

Silicon Carbide Reactor

Commercial 1,2-dichloro-1,1,2,2 tetrafluoroethane, containing 9% (molar) 1,1-dichloro-1,2,2,2 tetrafluoroethane, and hydrogen were fed at various feed rates over 38 hours into a straight tube silicon carbide reactor tube, as described above, operated at 0 psig. For a 12-hour period at 575°C and at an average time in synthesis of 14 hours, with a feed rate of 13.9 cc/min of hydrogen and 2 grams/hr of $CClF_2CClF_2$ (molar ratio $H_2/CClF_2CClF_2 = 2$); the average conversion of the $CClF_2CClF_2$ isomers was 35.6% with an average selectivity to $CHF_2CClF_2$ and its isomers of 28% and an average selectivity to $CHF_2CHF_2$ and its isomers of 27%.

EXAMPLE 32

$CF_2Cl_2 + H_2 \longrightarrow CF_2HCl + CH_2F_2$

Silicon Carbide Reactor

Dichlorodifluoromethane (2.6 g/hr) and hydrogen (molar ratio $H_2/CF_2Cl_2 = 1.0$) were fed into an empty 12.7 x 380 mm (1/2" x 15") silicon carbide straight tube reactor, as described above, over a 28-hour period. For four hours at 575°C during this run, at an average synthesis time of 26 hours; the average conversion of $CF_2Cl_2$ was 35.6%.

EXAMPLE 33

$CF_3CCl_3 + H_2 \longrightarrow CF_3CHCl_2 + CF_3CH_2Cl$

Hastelloy® Nickel Alloy Reactor

1,1,1-Trichloro-2,2,2-trifluoroethane and hydrogen were fed into an empty 380 x 9.5 mm (15" x 3/8") Hastelloy® C276 nickel alloy U-tube reactor, as described above, at 2070 kPa (300 psig) for a period of 28 hours. Over a 6-hour period at 425°C and 2070 kPa (300 psig), at an average time in synthesis of 17 hours, with a feed rate of $CF_3CCl_3$ of 6.25 g/hr and a hydrogen feed rate of 14.0 sccm (molar ratio $H_2/CF_3CCl_3 = 1.0$); the average conversion of $CF_3CCl_3$ was 33% with a selectivity to $CF_3CHCl_2$ of 95% and a selectivity to $CF_3CH_2Cl$ of 5%.

EXAMPLE 34

$CCl_4 + H_2 \longrightarrow CHCl_3 + CH_2Cl_2$

Inconel® Nickel Alloy Reactor

Carbon tetrachloride (6.57 g/hr) and hydrogen (200 sccm) were fed into an empty 380 x 6.35 mm (15" x 1/4") Inconel® 600 nickel alloy U-tube reactor, as described above, operated at pressures between 0 psig and 2070 kPa (300 psig) for 149 hours. For a 10-hour period at 457°C and 300 psig and an average time in synthesis of 136 hours, with a $CCl_4$ feed rate of 6.57 g/hr and a hydrogen feed rate of 200 sccm (molar ratio $H_2/CCl_4 = 12$); the average conversion of $CCl_4$ was 45% with an average selectivity to $CHCl_3$ of 59% and an average selectivity to $CH_2Cl_2$ of 2.8%.

EXAMPLE 35

$CF_3CHClF + H_2 \longrightarrow CF_3CH_2F$

High Hydrogen Ratio

2-Chloro-1,1,1,2-tetrafluoroethane and hydrogen were fed at varying rates for over 48 hours to a 1422 x 6.35 mm (56" x 1/4") Inconel® 600 nickel alloy coil reactor at 2070 kPa (300 psig) and temperatures between 550 and 600°C. For 4 hours at 600°C, at an average time in synthesis of 39 hours, with a $CF_3CHClF$ feed rate of 1.6 mL/hr and a $H_2$ feed rate of 130 sccm ($H_2/CF_3CHClF$ mol ratio = 20) the average conversion of $CF_3CHClF$ was 83% and the selectivity to $CF_3CH_2F$ was 94%. At a lower $H_2$ flow of 65 sccm and the same $CF_3CHClF$ feed rate and temperature ($H_2/CF_3CHClF$ mol ratio = 10) the average conversion of $CF_3CHClF$ over a 5-hour period was 90% with a 94% selectivity to $CF_3CH_2F$.

## EXAMPLE 36

$CF_3CHClF + H_2 \longrightarrow CF_3CH_2F$

High Hydrogen Ratio

2-Chloro-1,1,1,2-tetrafluoroethane and hydrogen were fed at various rates for 1207 hours into a 380 x 9.5 mm (15" x 3/8") Inconel® 600 nickel alloy U-tube, packed with 8.77 g 150 mesh nickel screen, and operated at various temperatures at 2070 kPa (300 psig). For a 23-hour period at 550°C and an average time in synthesis of 755 hours with a $CF_3CHClF$ feed rate of 0.4 ml/hour and a hydrogen feed rate of 18 sccm/min (molar ratio $H_2/CF_3CHClF = 11$), the average conversion of $CF_3CHCl$ was 99.6% with a selectivity to $CF_3CH_2F$ of 93%.

## EXAMPLE 37

$CCl_2FCF_3 + H_2 \longrightarrow CHClFCF_3 + CH_2FCF_3$

High Hydrogen Ratio

1,1-Dichloro-1,2,2,2-tetrafluoroethane and hydrogen were fed at various rates for 237 hours into a 380 x 9.5 mm (15" x 3/8") Hastelloy® C nickel alloy U-tube packed with 9.29 g of Conoco shot coke and operated at various temperatures at 2070 kPa (300 psig). For a 5-hour period at 575°C and an average time in synthesis of 227 hours with a $CCl_2FCF_3$ feed rate of 36 mL/hr and a hydrogen feed rate of 50 sccm/min (molar ratio $H_2/CCl_2FCF_3 = 40$), the average conversion of $CCl_2FCF_3$ was 100%. The selectivity to $CHClFCF_3$ was 32% and the selectivity to $CH_2FCF_3$ was 59%.

## EXAMPLE 38

$CF_3CClF_2 + H_2 \longrightarrow CF_3CHF_2$

2-Chloro-1,1,1,2,2-pentafluoroethane and hydrogen were fed at various rates to 380 x 9.5 mm (15" x 3/8") Hastelloy® C276 nickel alloy U-tube operated at 2070 kPa (300 psig) and various temperatures for 136 hours. For 10 hours, at an average time in synthesis of 58 hours and a temperature of 575°C, with a $CF_3CClF_2$ feed rate of 2.1 g/hr and a hydrogen feed rate of 14.0 sccm (molar ratio $H_2/CF_3CClF_2 = 2.5$), the conversion of $CF_3CClF_2$ was 89.5% and the selectivity to $CF_3CHF_2$ was 99.9%.

For an 8-hour period at an average time in synthesis of 131 hours and a temperature of 575°C, with a feed rate of $CF_3CClF_2$ of 4.15 g/hr and a hydrogen feed rate of 329 sccm (molar ratio $H_2/CF_3CClF_2 = 30$), the average conversion of $CF_3CClF_2$ was 39% with a selectivity to $CF_3CHF_2$ was 99.6%.

## Claims

1. A process for the hydrogenolysis of halocarbons comprising: contacting a halocarbon of the formula

$$C_nH_mF_pX_q$$

wherein
   X is Cl or Br,
   n is 1 to 10,
   m is 0 to 20,
   p is 0 to 21,
   q is 1 to 22, provided that $m+p+q$ equals $2n+2$ when the compound is acyclic and equals $2n$ when the compound is cyclic, and provided that when n is 1, q is at least 2, with at least 0.1 mole of hydrogen per mole of said halocarbon to effect hydrogenolysis of the halocarbon characterised in that a mixture of hydrogen and said halocarbon is contacted in a reaction vessel of aluminum, molybdenum, titanium, nickel, iron, cobalt, or their alloys or of chromium or of silicon carbide, which is either empty or is packed with particles or formed shapes of aluminum, molybdenum, titanium, nickel, iron, cobalt, or their alloys or silicon carbide or low surface area carbon at a pressure within the range of from 0 to

EP 0 495 892 B1

6900 kPa (0 psig to 1000 psig), at a temperature within the range of from 350°C to 700°C and for a time sufficient to produce as the major product a product of the above formula where at least one of X has been replaced by a hydrogen atom.

2. The process of claim 1 wherein the temperature ranges from 400°C to 700°C.

3. The process of claim 1 or 2 wherein the pressure is 0 to 3450 kPa (0 to 500 psig).

4. The process of any one of the preceding claims wherein the pressure is at least 172 kPa (25 psig).

5. The process of any one of the preceding claims wherein the reactor is empty.

6. The process of any one of the preceding claims wherein X is Cl, n is 1 to 4, m is 0 to 8, p is 0 to 9 and q is 1 to 9.

7. The process of claim 6 wherein n is 2 or 3.

8. The process of any one of claims 1 to 5 wherein the halocarbon is selected from $CF_3CCl_2F$, $CF_3CHClF$, $CCl_4$, $CCl_2F_2$, $CClF_2CClF_2$, $CHF_2CClF_2$, $C_2F_5Cl$, $CClF_2CCl_2F$, $CF_3CCl_3$, $CCl_2FCCl_2F$, $CClF_2CCl_3$ or $C_3ClF_7$.

9. The process of any one of the preceding claims wherein the ratio of moles of hydrogen to moles of halocarbon in said reaction vessel is from 0.2 to 40.

10. The process of any one of the preceding claims wherein the reaction time is between 0.2 and 8 minutes.

11. The process of any one of the preceding claims wherein the reaction vessel is nickel or a nickel alloy and is either empty or packed with nickel or a nickel alloy.

12. The process of any one of claims 1 to 10 wherein the reaction vessel is either an alloy containing 76 percent nickel, 15.5 percent chromium and 8 percent iron or an alloy containing 59 percent nickel, 15.5 percent chromium, 16 percent molybdenum and 3.75 percent tungsten; and wherein the reaction vessel either is empty or is packed with an alloy containing 76 percent nickel, 15.5 percent chromium and 8 percent iron, or of an alloy containing 59 percent nickel, 15.5 percent chromium, 16 percent molybdenum and 3.75 percent tungsten.

13. The process of any one of the preceding claims wherein q is 2 to 22, and the halocarbon and the hydrogen are contacted for a time such that the major product of the conversion is the hydrogenolysis product wherein just one X has been replaced by a hydrogen atom.

**Patentansprüche**

1. Verfahren zur Hydrogenolyse von Halogenkohlen(wasser)stoffen, umfassend das In-Berührung-Bringen eines Halogenkohlen(wasser)stoffs der Formel

$C_nH_mF_pX_q$ ,

in der
X Cl oder Br ist,
n 1 bis 10 ist,
m 0 bis 20 ist,
p 0 bis 21 ist,
q 1 bis 22 ist,
mit der Maßgabe, daß m + p + q gleich 2n + 2 ist, wenn die Verbindung acyclisch ist, und gleich 2n ist, wenn die Verbindung cyclisch ist, und mit der Maßgabe, daß dann, wenn n 1 ist, q wenigstens 2 ist, mit wenigstens 0,1 mol Wasserstoff auf 1 mol des Halogenkohlen(wasser)stoffs, um eine Hydrogenolyse des Halogenkohlen(wasser)stoffs zu bewirken,

13

dadurch gekennzeichnet, daß eine Mischung aus Wasserstoff und dem Halogenkohlen(wasser)stoff in Berührung gebracht wird in einem Reaktionsgefäß aus Aluminium, Molybdän, Titan, Nickel, Eisen, Cobalt oder deren Legierungen oder aus Chrom oder aus Siliciumcarbid, das entweder leer oder mit Teilchen oder Formkörpern aus Aluminium, Molybdän, Titan, Nickel, Eisen, Cobalt oder deren Legierungen oder Siliciumcarbid oder Kohlenstoff mit niedriger spezifischer Oberfläche gepackt ist, bei einem Überduck innerhalb des Bereichs von 0 bis 6 900 kPa (0 psig bis 1 000 psig) bei einer Temperatur von 350 °C bis 700 °C und für eine Zeitdauer, die ausreicht, um als Hauptprodukt ein Produkt der obigen Formel zu erzeugen, in der wenigstens einer der Substituenten X durch ein Wasserstoff-Atom ersetzt worden ist.

2. Verfahren nach Anspruch 1, worin die Temperatur im Bereich von 400 °C bis 700 °C liegt.

3. Verfahren nach Anspruch 1 oder 2, worin der Überdruck 0 bis 3 450 kPa (0 bis 500 psig) beträgt.

4. Verfahren nach irgendeinem der vorhergehenden Ansprüche, worin der Überdruck wenigstens 172 kPa (25 psig) beträgt.

5. Verfahren nach irgendeinem der vorhergehenden Ansprüche, worin der Reaktor leer ist.

6. Verfahren nach irgendeinem der vorhergehenden Ansprüche, worin X Cl ist, n 1 bis 4 ist, m 0 bis 8 ist, p 0 bis 9 ist und q 1 bis 9 ist.

7. Verfahren nach Anspruch 6, worin n 2 oder 3 ist.

8. Verfahren nach irgendeinem der Ansprüche 1 bis 5, worin der Halogenkohlen(wasser)stoff aus $CF_3CCl_2F$, $CF_3CHClF$, $CCl_4$, $CCl_2F_2$, $CClF_2CClF_2$, $CHF_2CClF_2$, $C_2F_5Cl$, $CClF_2CCl_2F$, $CF_3CCl_3$, $CCl_2FCCl_2F$, $CClF_2CCl_3$ oder $C_3ClF_7$ ausgewählt ist.

9. Verfahren nach irgendeinem der vorhergehenden Ansprüche, worin das Verhältnis der Stoffmenge (mol) Wasserstoffs zu der Stoffmenge (mol) des Halogenkohlen(wasser)stoffs in dem Reaktionsgefäß 0,2 bis 40 beträgt.

10. Verfahren nach irgendeinem der vorhergehenden Ansprüche, worin die Reaktionszeit zwischen 0,2 und 8 min liegt.

11. Verfahren nach irgendeinem der vorhergehenden Ansprüche, worin das Reaktionsgefäß aus Nickel oder einer Nickel-Legierung ist und entweder leer oder mit Nickel oder einer Nickel-Legierung gepackt ist.

12. Verfahren nach irgendeinem der Ansprüche 1 bis 10, worin das Reaktionsgefäß entweder aus einer Legierung, die 76 % Nickel, 15,5 % Chrom und 8 % Eisen enthält, oder aus einer Legierung, die 59 % Nickel, 15,5 % Chrom, 16 % Molybdän und 3,75 % Wolfram enthält, besteht und entweder leer ist oder mit einer Legierung, die 76 % Nickel, 15,5 % Chrom und 8 % Eisen enthält, oder mit einer Legierung, die 59 % Nickel, 15,5 % Chrom, 16 % Molybdän und 3,75 % Wolfram enthält, gepackt ist.

13. Verfahren nach irgendeinem der vorhergehenden Ansprüche, worin q 2 bis 22 ist und der Halogenkohlen(wasser)stoff und der Wasserstoff eine solche Zeit in Berührung gebracht werden, daß das Hauptprodukt der Umwandlung das Hydrogenolyse-Produkt ist, wo genau ein X durch ein Wasserstoff-Atom ersetzt worden ist.

**Revendications**

1. Procédé d'hydrogénolyse d'halogénocarbures comprenant : la mise en contact d'un halogénocarbure de formule

$C_nH_mF_pX_q$

dans laquelle
    X est Cl ou Br,

n a une valeur de 1 à 10

m a une valeur de 0 à 20,

p a une valeur de 0 à 21,

q a une valeur de 1 à 22, à condition que m+p+q soit égal à 2n+2 quand le composé est acyclique et soit égal à 2n quand le composé est cyclique, et à condition que quand n est égal à 1, q soit au moins égal à 2, avec au moins 0,1 mole d'hydrogène par mole dudit halogénocarbure pour effectuer l'hydrogénolyse de l'halogénocarbure caractérisé en ce qu'un mélange d'hydrogène et dudit halogénocarbure est mis en contact dans un réacteur en aluminium, molybdène, titane, nickel, fer, cobalt, ou leurs alliages ou en chrome ou en carbure de silicium, qui est soit vide soit garni de particules ou de formes façonnées en aluminium, molybdène, titane, nickel, fer, cobalt, ou leurs alliages ou en carbure de silicium ou en carbone de faible surface spécifique, à une pression relative dans l'intervalle de 0 à 6900 kPa, à une température dans l'intervalle de 350°C à 700°C et pendant un temps suffisant pour donner, comme produit principal, un produit ayant la formule ci-dessus dans laquelle au moins un des X a été remplacé par un atome d'hydrogène.

2. Procédé de la revendication 1 dans lequel la température est dans l'intervalle de 400°C à 700°C.

3. Procédé selon l'une quelconque des revendications 1 et 2 dans lequel la pression est de 0 à 3450 kPa.

4. Procédé selon l'une quelconque des revendications précédentes dans lequel la pression est au moins égale à 172 kPa.

5. Procédé selon l'une quelconque des revendications précédentes dans lequel le réacteur est vide.

6. Procédé selon l'une quelconque des revendications précédentes dans lequel X est Cl, n a une valeur de 1 à 4, m a une valeur de 0 à 8, p a une valeur de 0 à 9 et q a une valeur de 1 à 9.

7. Procédé de la revendication 6 dans lequel n est 2 ou 3.

8. Procédé selon l'une quelconque des revendications précédentes dans lequel l'halogénocarbure est choisi parmi $CF_3CCl_2F$, $CF_3CHClF$, $CCl_4$, $CCl_2F_2$, $CClF_2CClF_2$, $CHF_2CClF_2$, $C_2F_5Cl$, $CClF_2CCl_2F$, $CF_3CCl_3$, $CCl_2FCCl_2F$, $CClF_2CCl_3$ et $C_3ClF_7$.

9. Procédé selon l'une quelconque des revendications précédentes dans lequel le rapport du nombre de moles d'hydrogène au nombre de moles d'halogénocarbure dans ledit réacteur va de 0,2 à 40.

10. Procédé selon l'une quelconque des revendications précédentes dans lequel le tempos de réaction est compris entre 0,2 et 8 minutes.

11. Procédé selon l'une quelconque des revendications précédentes dans lequel le réacteur est en nickel ou en un alliage de nickel et est soit vide soit rempli de nickel ou d'un alliage de nickel.

12. Procédé selon l'une quelconque des revendications 1 à 10 dans lequel le réacteur est soit un alliage contenant 76 pour cent de nickel, 15,5 pour cent de chrome et 8 pour cent de fer, soit un alliage contenant 59 pour cent de nickel, 15,5 pour cent de chrome, 16 pour cent de molybdène et 3,75 pour cent de tungstène; et dans lequel le réacteur est soit vide soit garni d'un alliage contenant 76 pour cent de nickel, 15,5 pour cent de chrome et 8 pour cent de fer, ou d'un alliage contenant 59 pour cent de nickel, 15,5 pour cent de chrome, 16 pour cent de molybdène et 3,75 pour cent de tungstène.

13. Procédé selon l'une quelconque des revendications précédentes dans lequel q a une valeur de 2 à 22, et l'halogénocarbure et l'hydrogène sont mis en contact pendant un temps tel que le produit principal de la conversion est le produit d'hydrogénolyse dans lequel seul un X a été remplacé par un atome d'hydrogène.